# EUROPEAN PATENT SPECIFICATION

(11) **EP 3 374 031 B1**
(45) Date of publication and mention of the grant of the patent: **05.08.2020**
(21) Application number: 16727485.1
(22) Date of filing: 07.06.2016
(51) Int. Cl.: A61Q 11/00, A61K 8/25, A61K 8/29, A61K 8/73

(54) **ORAL CARE COMPOSITION**
MUNDPFLEGEZUSAMMENSETZUNG
COMPOSITION DE PROTECTION ORALE

(30) Priority: 10.11.2015 WO PCT/CN2015/094184; 08.12.2015 EP 15198434
(43) Date of publication of application: 19.09.2018
(73) Proprietor: UNILEVER N.V., 3013 AL Rotterdam (NL); Unilever PLC, London Greater London EC4Y 0DY (GB)
(72) Inventor: LI, Yajuan, Shanghai 200335 (CN); TANG, Xuezhi, Shanghai 200335 (CN); WANG, Jinfang, Shanghai 200335 (CN)
(74) Representative: Tansley, Sally Elizabeth
(86) International application number: PCT/EP2016/062847
(87) International publication number: WO 2017/080687

(56) References cited:
- EP-A2- 2 349 189
- WO-A1-2010/105922
- WO-A1-2012/078136
- WO-A1-2014/124904
- WO-A1-2015/036277
- WO-A1-2015/036285
- WO-A2-2012/078337
- US-A1- 2010 189 663

## Description

### Technical Field of the Invention

The present invention relates to oral care compositions such as tooth pastes, gums, and mouthwashes. In particular the present invention relates to an oral care composition containing calcium silicate, particulate benefit agents and a deposition aid. The invention also relates to the use of such composition for benefiting teeth of an individual.

### Background of the Invention

Many products we consume have a negative impact on our teeth and mouth. Acidic drinks and sweets, for example, can result in tooth erosion by attacking enamel which is the outer coating that protects the teeth. Moreover, tobacco based products as well as beverages like coffee and tea can stain or reduce the whiteness of one's teeth. These staining and discoloring substances are often able to permeate the enamel layer. This problem occurs gradually over many years, but imparts a noticeable discoloration of the enamel of one's teeth.

Consumers have always had a strong desire for healthy and white teeth. Benefit agents such as coloring agents, biomineralization agents and antibacterial agents are commonly incorporated in oral care compositions to be delivered to teeth to provide benefits such as whitening, remineralization and better oral hygiene. Therefore, it is always desired to improve the delivery efficiencies of benefit agents to maximize the effectiveness of such benefit agents.

However, it is usually difficult to achieve high delivery of benefit agents from oral care compositions. Those benefit agents do not strongly adhere to the tooth surfaces which makes them easy to be rinsed off during daily oral hygiene routine like brushing teeth.

The present inventors have now recognized there is a need to develop an oral care composition that can enhance the delivery efficiency of benefit agents. It has been found unexpectedly that deposition of calcium silicate and/or benefit agents on tooth surfaces can be enhanced by using calcium silicate in combination with xanthan gum as deposition aid.

### Additional Information

WO 2008/068149 A (Unilever) discloses an oral care product comprising a first composition comprising an insoluble calcium salt that is not a calcium phosphate salt, a second independent composition comprising a source of phosphate ions, and a means for delivering each of the compositions to the surface of the teeth. The preferred insoluble calcium salt is calcium silicate.

WO 2012/031785 A (Unilever) discloses composite particle actives suitable for use in oral care compositions. The composite particle actives have a core and a coating whereby the coating interacts with phosphate ions to produce calcium and phosphate reaction products that are suitable to adhere to tooth enamel and/or dentin in order to improve characteristics of teeth.

WO 2012/031786 A (Unilever) discloses oral care compositions with composite particle actives described in WO 2012/031785 A (Unilever).

None of the additional information above describes an oral care composition comprising calcium silicate and xanthan gum as deposition aid, and particularly that such composition can enhance the deposition of calcium silicate and/or benefit agents on tooth surfaces.

### Tests and Definitions

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purpose of the present invention means a paste or gel dentifrice for use with a toothbrush. Especially preferred are tooth pastes suitable for cleaning teeth by brushing for about two minutes.

### Mouth Wash

"Mouth wash" for the purpose of the present invention means liquid dentifrice for use in rinsing the mouth. Especially preferred are mouth washes suitable for rinsing the mouth by swishing and/or gargling for about half a minute before expectorating.

### Particle Size

"Particle size" for the purpose of the present invention means D50 particle size. The D50 particle size of a particulate material is the particle size diameter at which 50 wt% of the particles are larger in diameter and 50 wt% are smaller in diameter. For the purpose of the present invention, particle sizes and distribution are measured using Malvern Mastersizer 2000 and Malvern ZetaSizer Nano series.

### Composite Particle

"Composite particle" for the purpose of the present invention means a particle comprising a first component core and a second component coating wherein the core and coating are composed of different materials.

### Deposition Aid

"Deposition aid" for the purpose of the present invention means a material which aids deposition of actives such as calcium silicate and/or benefit agents from the continuous phase of the composition onto the tooth surfaces during use of the composition.

### Refractive Index

Refractive index is quoted at a temperature of 25°C and a wavelength of 589 nm.

### pH

pH is quoted at atmospheric pressure and a temperature of 25°C. When referring to the pH of an oral care composition, this means the pH measured when 5 parts by weight of the composition is uniformly dispersed and/or dissolved in 20 parts by weight pure water at 25°C. In particular the pH may be measured by manually mixing 5 g oral care composition with 20 mL water for 30 s, then immediately testing the pH with indicator or a pH meter.

### Solubility

"Soluble" and "insoluble", as used herein, refers to the solubility of a source (e.g., like calcium salts) in water at 25°C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Sparingly soluble", therefore, is defined to mean a source that dissolves in water to give a solution with a concentration of greater than 0.001 moles per litre and less than 0.1 moles per litre.

### Water of Hydration

"Water of hydration" for the purpose of the present invention means water chemically combined with a substance in a way that it can be removed by heating without substantially changing the chemical composition of the substance. In particular, water which could only be removed when heated above 200°C. The water loss is measured using thermo gravimetric analysis (TGA) with a Netzsch TG instrument. The TGA is conducted under an N₂ atmosphere with heating rate of 10 degree/min in the range of 30 to 900°C.

### Substantially Free

"Substantially free of" for the purpose of the present invention means less than 1.5%, and preferably less than 1.0%, and more preferably less than 0.75% and more preferably still less than 0.5%, and even more preferably less than 0.1% and most preferably from 0.0 to 0.01 % by weight, based on total weight of the oral care composition, including all ranges subsumed therein.

### Dual-Phase

"Dual-Phase" for the purpose of the present invention means a composition having two independent phases which are physically separate.

### Viscosity

Viscosity of a tooth paste is the value taken at room temperature (25°C) with a Brookfield Viscometer, Spindle No.4 and at a speed of 5 rpm. Values are quoted in centipoises (cP=mPa.s) unless otherwise specified.

### Remineralization

"Remineralization" for the purpose of the present invention means *in situ* (i.e. in the oral cavity) generation of calcium phosphate on teeth (including layers on teeth from 10 nm to 20 microns, and preferably from 75 nm to 10 microns, and most preferably, from 150 nm to 5 microns thick including all ranges subsumed therein) to reduce the likelihood of tooth sensitivity, tooth decay, regenerate enamel and/or improve the appearance of teeth by whitening through the generation of such new calcium phosphate.

### Miscellaneous

All amounts are by weight of the final oral care composition, unless otherwise specified.

It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

Where a feature is disclosed with respect to a particular aspect of the invention (for example a composition of the invention), such disclosure is also to be considered to apply to any other aspect of the invention (for example a method of the invention) *mutatis mutandis.*

### Summary of the Invention

In a first aspect, the present invention is directed to an oral care composition comprising:
a) from 5 to 80% by weight of calcium silicate;
b) from 0.4% to 2% by weight of xanthan gum;
c) a benefit agent; and
d) a physiologically acceptable carrier;
   wherein the benefit agent is selected from coloring agents, biomineralization agents, antibacterial agents or mixtures thereof and wherein the biomineralization agents is amorphous calcium phosphate, α-tricalcium phosphate, β-tricalcium phosphate; calcium carbonate, calcium deficient hydroxyapatite (Ca₉(HPO₄)(PO₄)₅OH), dicalcium phosphate (CaHPO₄), dicalcium phosphate dehydrate (CaHPO₄·2H₂O), hydroxyapatite (Ca₁₀(PO₄)₆(OH)₂), monocalcium phosphate monohydrate (Ca(H₂PO₄)₂·H₂O), octacalcium phosphate (Ca₈H₂(PO₄)₆·5H₂O) and tetracalcium phosphate (Ca₄(PO₄)₂O) or mixtures thereof.

In a second aspect, the present invention is directed to a packaged oral care product comprising the oral care composition of the first aspect of this invention.

In a fourth aspect, the present invention is directed to the use of xanthan gum in the oral care composition of any embodiment of the first aspect for enhancing the deposition of calcium silicate and/or benefit agents on tooth surfaces.

All other aspects of the present invention will more readily become apparent upon considering the detailed description and examples which follow.

### Detailed Description

Now it has been found unexpectedly that an oral care composition comprising calcium silicate, and xanthan gum as a deposition aid can enhance the delivery efficiency of benefit agents to tooth surfaces, providing improved tooth benefits. Delivery efficiency as used herein means the ability to deliver and deposit benefit agents to tooth surfaces of an individual.

### CALCIUM SILICATE

In a preferred embodiment, the calcium silicate used is CaSiO₃ which has low water solubility and is made commercially available under the name Sorbosil CA40 from PQ Corporation. In another preferred embodiment, the calcium silicate is insoluble, present as the composite material calcium oxide-silica (CaO-SiO₂), which is described, for example, in international patent application published as WO 2008/01517(Unilever).

For a calcium silicate composite material, the atom ratio of calcium to silicon (Ca:Si) may be from 1:30 to 3:1. The Ca:Si ratio is preferably 1:20 to 2:1, and more preferably, from 1:10 to 1:1, and most preferably, from about 1:7 to 1:1.5. The calcium silicate may comprise mono-calcium silicate, bi-calcium silicate, or tri-calcium silicate. The calcium silicate may be in a crystalline or amorphous state or even in a mesoporous state.

In addition to calcium oxide, silica, the particles comprising the calcium silicate which is not hydrated may comprise other components, such as metal cations, anions (such as phosphate).

However, it is preferred that the particles comprise calcium oxide, silica in an amount of at least 70% by weight of the particles, more preferably at least 80%, more preferably still at least 90% and even more preferably at least 95%. Most preferably the particles consist of (or at least consist essentially of) calcium oxide, silica.

In an especially preferred embodiment, the calcium silicate is calcium silicate hydrate. The calcium silicate hydrate for use in the present invention comprises at least calcium oxide (CaO), silica (SiO₂) and water. Compared with conventional calcium silicate which are not hydrated, the calcium silicate hydrate comprises the water of hydration in an amount of at least 5% by weight of the calcium silicate hydrate, preferably at least 10%, more preferably at least 15%, even more preferably at least 20% and most preferably at least 25%. The water content is typically no greater than 50% by weight of the calcium silicate hydrate, more preferably no greater than 40%, even more preferably no greater than 35% and most preferably no greater than 30%.

The calcium silicate hydrate preferably comprises at least 20% silica by weight of the calcium silicate hydrate, more preferably at least 30%, more preferably still at least 40% and most preferably at least 55%. The silica content is preferably no greater than 70% by weight of the calcium silicate hydrate, more preferably no greater than 65% and most preferably no greater than 60%.

To provide calcium necessary for remineralization, the calcium silicate hydrate preferably comprises calcium oxide in an amount of at least 5% by weight of the calcium silicate hydrate, more preferably at least 7%, more preferably still at least 10%, even more preferably at least 12% and most preferably at least 15%. The calcium oxide content is typically no greater than 50% by weight of the calcium silicate hydrate, more preferably no greater than 40%, even more preferably no greater than 30% and most preferably no greater than 25%.

The calcium silicate hydrate preferably comprises Ca and Si in an atom ratio (Ca:Si) less than 1:1, more preferably less than 1:1.2, more preferably still from 1:1.5 to 1:4 and most preferably from 1:1.7 to 1:3.

The calcium silicate may be amorphous or at least partly crystalline or mesoporous. The calcium silicate is preferably particulate as this allows for maximum surface area for contact with dental tissue. Thus preferably the composition comprises particles comprising the calcium silicate. Preferably from 10 to 100%, and especially, from 25 to 100%, and most especially, from 70% to 1 00% by weight of the particles comprising calcium silicate used in this invention have a particle size from 100 nm to less than 50 microns, preferably from 500 nm to 30 microns, more preferably from 1 micron to 20 microns, most preferably from 3 micron to 15 microns.

In addition to calcium oxide, silica and water, the particles which comprise the calcium silicate hydrate may comprise other components, such as metal cations, anions (such as phosphate). However, it is preferred that the particles comprise CaO, SiO₂ and water in an amount of at least 70% by weight of the particles, more preferably at least 80%, more preferably still at least 90% and even more preferably at least 95%. Most preferably the particles consist of (or at least consist essentially of) CaO, SiO₂ and water. The oral care composition of the present invention comprises from 5 to 80% by weight of the calcium silicate, more preferably from 5 to 50%, most preferably from 8 to 30%, based on the total weight of the oral care composition and including all ranges subsumed therein.

### DEPOSITION AID

The deposition aid suitable for use in this invention is limited only to the extent that the same may be used in the mouth. The deposition aid is a natural gum, and is xanthan gum. Xanthan gum is an extracellular polysaccharide secreted by the microorganism *Xanthomonas campestris.* It is a linear (1, 4)-linked β-D-glucose backbone (as in cellulose) with a trisaccharide side chain on every other glucose at C-3. This side chain comprises a glucuronic acid residue linked (1, 4) to a terminal mannose unit and (1, 2) to a second mannose that connects to the backbone. Approximately 50% of the terminal mannose residues are pyruvated and the non-terminal residue usually carries an acetyl group at C-6.

The structure of xanthan gum is given below: 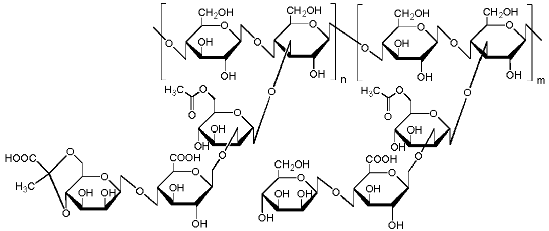 where:
n and m are integers from 0 to 10,000, and the sum of n+m ranges from 10 to 10,000, preferably from 100 to 8,000, more preferably from 500 to 5,000, most preferably from 1,000 to 3,000.

The xanthan gum derivatives are prepared by reacting non-derivatized gum with quaternary ammonium compounds in an amount equal to or greater than the stoichiometric amount required for complete derivatization. The quaternary ammonium compounds are preferably having a single alkyl or alkenyl substituent containing from 13 to 24 carbon atoms, and/or two alkyl or alkenyl substituents of 12 to 24 carbon atoms per substituent. Most preferably, the quaternary ammonium compound is alkyl dimethylbenzylammonium chloride with an alkyl group containing from 13 to 24 carbon atoms. The oral care composition of the present invention comprises from 0.4 to 2% by weight of xanthan gum, more preferably from 0.5 to 1.5%, most preferably from 0.6 to 1.2%, based on the total weight of the oral care composition and including all ranges subsumed therein.

### BENEFIT AGENT

Benefit agents as used herein means an active typically delivered to human teeth and/or the oral cavity including the gums to enhance or improve a characteristic of those dental tissues. The only limitation with respect to the benefit agents that may be used in this invention is that the same is suitable for use in the mouth. Typically the benefit agents include coloring agents, biomineralization agents, antibacterial agents or mixtures thereof. For example, coloring agent such as particulate whitening agents and pigments, preferably a particulate whitening agent. Preferably, the pigment, when used, is violet or blue having a hue angle, h, in the CIELAB system of from 220 to 320 degrees. These pigments may be selected from one or more of those listed in the Colour Index International, listed as pigment blue 1 through to pigment blue 83, and pigment violet 1 through to pigment violet 56; biomineralization agents for tooth enamel remineralization may be one or more of amorphous calcium phosphate, α-tricalcium phosphate, β-tricalcium phosphate; calcium carbonate, calcium deficient hydroxyapatite (Ca₉(HPO₄)(PO₄)₅OH), dicalcium phosphate (CaHPO₄), dicalcium phosphate dehydrate (CaHPO₄ • 2H₂O), hydroxyapatite (Ca₁₀(PO₄)₆(OH)₂), monocalcium phosphate monohydrate (Ca(H₂PO₄)₂ • H₂O), octacalcium phosphate (Ca₈H₂(PO₄)₆ • 5H₂O) and tetracalcium phosphate (Ca₄(PO₄)₂O); antibacterial agents may be selected from one or more of metal salts where the metal is selected from zinc, copper, silver or a mixture thereof, triclosan, triclosan monophosphate, triclocarban, curcumin, quaternary ammonium compounds, bisbiguanides and long chain tertiary amines, preferably zinc salts including zinc oxide, zinc chloride, zinc acetate, zinc ascorbate, zinc sulphate, zinc nitrate, zinc citrate, zinc lactate, zinc peroxide, zinc fluoride, zinc ammonium sulfate, zinc bromide, zinc iodide, zinc gluconate, zinc tartarate, zinc succinate, zinc formate, zinc phenol sulfonate, zinc salicylate, zinc glycerophosphate or a mixture thereof.

The benefit agent is preferably particulate as this allows for maximum surface area for contact with dental tissue.

In a preferred embodiment, the benefit agent is a particulate whitening agent for tooth whitening.

Typically, the particulate whitening agent comprises a material suitable to physically and immediately improve characteristics of teeth and especially whiten teeth. In order to provide excellent whitening effect, the material is preferred to have a high refractive index of at least 1.9, more preferably at least 2.0, even more preferably at least 2.2, even more preferably still at least 2.4 and most preferably at least 2.5. The maximum refractive index of the material is not particularly limited but preferably up to 4.0. Preferably, the material has a refractive index ranging from 1.9 to 4.0.

Particularly suitable materials are metal salts and preferred are salts where the metal is selected from zinc (Zn), titanium (Ti), zirconium (Zr) or a combination thereof. Preferably, the metal salts is (or at least comprises) a metal oxide such as titanium dioxide (TiO₂), zinc oxide (ZnO), zirconium dioxide (ZrO₂) or a combination thereof. In addition, the particulate whitening agent can also comprise non-metal oxides such as silicon monoxide (SiO).

In a preferred embodiment, the particulate whitening agent comprises metal oxides, non-metal oxides or a combination thereof in an amount of at least 50% by weight of the whitening agent and more preferably at least 70%, more preferably still from 80 to 100% and most preferably from 85 to 95%. In an especially preferred embodiment, the particulate whitening agent is at least 50% by weight titanium dioxide, and most preferably, from 60 to 100% by weight titanium dioxide, based on total weight of the whitening agent and including all ranges subsumed therein. In another especially preferred embodiment, the particulate whitening agents are slightly soluble or insoluble in water, but most preferably, insoluble in water.

In a preferred embodiment, the particulate whitening agents are composite particles. The refractive index of a composite particle comprising more than one material can be calculated based on the refractive indices and volume fractions of the constituents using effective medium theory, as is described for example in WO 2009/023353.

The composite particle comprises a first component core and a second component coating. Typically, the core of the composite particle comprises a material suitable to physically and immediately improve characteristics of teeth and especially whiten teeth. In order to provide excellent whitening effect, the material is preferred to have a high refractive index of at least 1.9, more preferably at least 2.0, even more preferably at least 2.2, even more preferably still at least 2.4 and most preferably at least 2.5. The maximum refractive index of the material is not particularly limited but preferably up to 4.0. Preferably, the material has a refractive index ranging from 1.9 to 4.0.

Particular suitable materials are metal salts and preferred are salts where the metal is selected from zinc (Zn), titanium (Ti), zirconium (Zr) or a combination thereof. Preferably, the metal salt is (or at least comprises) a metal oxide such as titanium dioxide (TiO₂), zinc oxide (ZnO), zirconium dioxide (ZrO₂) or a combination thereof. In addition, the core of the composite particle can also comprise non-metal oxides such as silicon monoxide (SiO).

The core of the composite particle typically makes up from 3 to 98%, and preferably from 6 to 65%, and most preferably from 10 to 55% by weight of the composite particle, based on total weight of the composite particle and including all ranges subsumed therein. In a preferred embodiment, the core comprises metal oxides, non-metal oxides or a combination thereof in an amount of at least 50% by weight of the core and more preferably at least 70%, more preferably still from 80 to 1 00% and most preferably from 85 to 95%. In an especially preferred embodiment, the core is at least 50% by weight titanium dioxide, and most preferably, from 60 to 100% by weight titanium dioxide, based on total weight of the first component core.

The second component coating comprises material suitable to adhere to tooth enamel, dentin or both. Typically the coating material comprises the element calcium, and optionally, other metals like potassium, sodium, aluminium, magnesium as well as mixtures thereof whereby such optional metals are provided as, for example, sulphates, lactates, oxides, carbonates or silicates. Optionally, the coating material may be aluminium oxide or silica. In a preferred embodiment, the coating material is suitable to provide a biological or chemical improvement to teeth which is long term (e.g., results in hydroxyapatite formation). Preferably, the coating employed comprises at least 50% by weight elemental calcium, and most preferably, at least 65% by weight elemental calcium based on total weight of metal in the coating. In an especially preferred embodiment, the metal in the coating is from 80 to 100% by weight of elemental calcium, based on total weight of metal in the second component coating and including all ranges subsumed therein. In another especially preferred embodiment, the core and the coating are slightly soluble or insoluble in water, but most preferably, insoluble in water.

In an especially desired embodiment, the second component coating can comprise, for example, calcium phosphate, calcium gluconate, calcium oxide, calcium lactate, calcium carbonate, calcium hydroxide, calcium sulphate, calcium carboxymethyl cellulose, calcium alginate, calcium salts of citric acid, calcium silicate, or mixture thereof. In another desired embodiment, the calcium source in the coating comprises calcium silicate.

In yet another preferred embodiment, the coating can comprise the element calcium which originates from insoluble calcium silicate, present as the composite material calcium oxide-silica (CaO-SiO₂) as described in international patent applications published as WO2008/015117 and WO 2008/068248.

When a calcium silicate composite material is employed as coating, the ratio of calcium to silicon (Ga:Si) may be from 1:10 to 3:1. The Ca:Si ratio is preferably from 1:5 to 2:1, and more preferably, from 1:3 to 2:1, and most preferably, from about 1:2 to 2:1. The calcium silicate may comprise mono-calcium silicate, bi-calcium silicate, or tri-calcium silicate whereby ratios of calcium to silicon (Ca:Si) should be understood to be atom ratios.

Usually, at least 30% of the outer surface area of the first component core is coated with the second component coating, preferably at least 50% of the core is coated with the coating, most preferably, from 70 to 100% of the outer surface area of the first component core is coated with the second component coating.

In an especially preferred embodiment, the particulate whitening agent is titanium dioxide coated with calcium silicate.

The particulate whitening agent according to the present invention can be of different sizes and shapes. The particles may be of spherical, platelet or irregular shape form. The diameter of the particulate whitening agent is often from 10 nm to less than 50 microns, and preferably, from 75 nm to less than 10 microns. In an especially preferred embodiment, the diameter of particles is from 100 nm to 5 microns, including all ranges subsumed therein. For composite particles, in a preferred embodiment, at least 40%, and preferably, at least 60%, and most preferably, from 75 to 99.5% of the diameter of the composite particle is the core, including all ranges subsumed therein.

The oral care composition of the present invention may comprise a single benefit agent or a mixture of two or more benefit agents. Typically, the benefit agent is present in an amount from 0.25 to 60%, and more preferably, from 0.5 to 40%, and most preferably, from 1 to 30% by total weight of the oral care composition and including all ranges subsumed therein.

The relative weight ratio of calcium silicate to benefit agent may range from 1:10 to 4:1, preferably from 1:5 to 3:1, most preferably from 1:3 to 2:1.

### OPTIONAL COMPONENTS

The oral care composition of the present invention is found to be capable of remineralization of teeth without inclusion of a phosphate source in the composition itself. Without wishing to be bound by theory the present inventors believe that this may be because the calcium silicate in the oral composition of the present invention reacts with phosphate ions in saliva and/or Si-OH groups may have an affinity for Ca ions in teeth. The xanthan gum acts as a deposition aid which enhances the deposition of calcium silicate and/or benefit agents onto the tooth surface. The remineralization of calcium silicate around the benefit agents further helps the retention of those benefit agents on tooth surfaces by enhancing their resistance to shear force.

Thus, in one embodiment, the composition may be substantially free of phosphate source.

For anhydrous compositions (i.e. compositions substantially free of water) or a dual phase hydrous compositions, it is preferred to compound a phosphate source in the composition.

The phosphate source that may be used in this invention is limited only to the extent that the same may be used in a composition suitable for use in the mouth. Illustrative examples of the types of phosphate source suitable for use in this invention include trisodium phosphate, monosodium dihydrogen phosphate, disodium hydrogen phosphate, sodium pyrophosphate, tetrasodium pyrophosphate, sodium hexametaphosphate, tripotassium phosphate, monopotassium dihydrogen phosphate, dipotassium hydrogen phosphate, or mixtures thereof. The phosphate source is preferably one which is water soluble.

When used, the phosphate source typically makes up from 0.5 to 22%, and preferably, from 2 to 18%, and most preferably, from 4 to 16% by weight of the oral care composition, based on total weight of the oral care composition and including all ranges subsumed therein. In a preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein.

The composition of the present invention is an oral care composition and typically comprises physiologically acceptable carrier. The carrier preferably comprises at least surfactant, thickener, humectant or a combination thereof.

Preferably the oral care composition comprises a surfactant. Preferably the composition comprises at least 0.01 % surfactant by weight of the composition, more preferably at least 0.1 % and most preferably from 0.5 to 7%. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is an anionic surfactant. The preferred anionic surfactants are sodium lauryl sulphate and/or sodium dodecylbenzene sulfonate. Most preferably the surfactant is sodium lauryl sulphate.

In an especially preferred embodiment, the xanthan gum also behaves as the only thickener in the oral care composition.

In another preferred embodiment, the oral care composition further comprises thickeners other than xanthan gum. Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, Irish moss, starch, modified starch, silica based thickeners including silica aerogels, magnesium aluminum silicate (e.g., Veegum), Carbomers (cross-linked acrylates) and mixtures thereof.

Typically, sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

Thickener typically makes up from 0.01 to about 1 0%, more preferably from 0.1 to 9%, and most preferably, from 1.5 to 8% by weight of the oral care composition, based on total weight of the composition and including all ranges subsumed therein.

When the oral care composition of this invention is a toothpaste or gel, the same typically has a viscosity from about 30,000 to 180,000 centipoise, and preferably, from 60,000 to 170,000 centipoise, and most preferably, from 65,000 to 165,000 centipoise.
Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. More preferably, the carrier humectant makes up from 25 to 80%, and most preferably, from 45 to 70% by weight of the composition, based on total weight of the composition and including all ranges subsumed therein.

The oral care composition of the present invention may contain a variety of other ingredients which are common in the art to enhance physical properties and performance. These ingredients include antimicrobial agents in addition to the antibacterial benefit agent which may be included in the composition, anti-inflammatory agents, anti-caries agents, plaque buffers, fluoride sources, vitamins, plant extracts, desensitizing agents, anti-calculus agents, biomolecules, flavors, proteinaceous materials, preservatives, opacifying agents, coloring agents in addition to the coloring benefit agent which may be included in the composition, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

The oral care composition of this invention can be used for providing benefits to teeth of an individual such as remineralization and/or sterilization and/or whitening comprising applying the composition to at least one surface of the teeth of the individual. The oral care composition of this invention may additionally or alternatively be for use as a medicament and/or used in the manufacture of a medicament for providing an oral care benefit as described herein, such as for whitening of the teeth of an individual. Alternatively and preferably, the use is non-therapeutic.

In a preferred embodiment, the oral care composition is a monophase anhydrous composition.

In another preferred embodiment, the oral care composition is a dual-phase composition comprising a calcium source and a phosphate source, wherein the calcium silicate is present in the calcium source and the xanthan gum can be presented in either of the two phases. The two phases are physically separate from one another by being in independent phases. The delivery of the two independent phases to the teeth may be simultaneous or sequential. In a preferred embodiment, the phases are delivered simultaneously.

Typically, the dual-phase composition is delivered by a dual-tube having a first compartment for the first phase comprising calcium source and a second compartment for the second phase comprising the phosphate source, which allows for co-extrusion of the two phases.

In a preferred embodiment, such a dual-tube has one of the compartments surrounding the other. In such embodiments, one phase is present as a sheath, surrounding the other phase in the core. In an especially preferred embodiment, the phase as the core comprises the calcium source and the sheath phase comprises the phosphate source.

In another preferred embodiment, such a dual-tube has the two compartments side by side within the same tube. In such embodiments, the two phases are extruded from the tube as one, such extrusion being termed "contact extrusion". A pump head may be used in such a dual-tube for squeezing the two phases from the tube as one.

The dual-phase oral care composition may be a gel composition that comprises two independent gel phases, the first comprising the calcium source and the second comprising the phosphate source. The means of delivery may involve a cotton rod, or a tray, onto which the calcium source and the phosphate source are applied, prior to the tray being placed in contact with the teeth.

Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area. In liquid mouthwash form the composition may be packaged in a bottle, sachet or other convenient container.

The composition can be effective even when used in an individual's daily oral hygiene routine. For example, the composition may be brushed onto the teeth and/or be rinsed around the inside of the mouth of the individual. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day. When the oral care composition is a dual-phase composition, the two phases of the composition are mixed during application. The mixed phases are typically left on the teeth for from 3 minutes to 10 hours, more preferably from 3 minutes to 8 hours. The application may be carried out daily.

The following examples are provided to facilitate an understanding of the present invention. The examples are not provided to limit the scope of the claims.

### Example 1

This example demonstrates the improved deposition of particles on tooth surfaces by using calcium silicate in combination with xanthan gum. All ingredients are expressed by weight percent of the total formulation, and as level of active ingredient.

**TABLE 1**

| | **Samples** | | | | | | |
|---|---|---|---|---|---|---|---|
| | **1** | **2** | **3** | **4** | **5** | **6** | **7** |
| Sorbitol | 45 | 45 | 45 | 45 | 45 | 45 | 45 |
| Water | 30 | 29 | 29 | 29 | 29 | 29 | 29 |
| PVM/MA Copolymer^{a} | - | 1 | - | - | - | - | - |
| Sodium Alginate^{b} | - | - | 1 | - | - | - | - |
| Xantham gum^{c} | - | - | - | 1 | - | - | - |
| Carrageenan^{d} | - | - | - | - | 1 | - | - |
| Acrylamide and sodium acrylamido-tertio-butyl sulfonate copolymer^{e} | - | - | - | - | - | 1 | - |
| Acrylates Copolymer^{f} | - | - | - | - | - | - | 1 |
| Titanium dioxide coated with calcium silicate^{g} | 15 | 15 | 15 | 15 | 15 | 15 | 15 |
| Calcium silicate^{h} | 10 | 10 | 10 | 10 | 10 | 10 | 10 |

| | | | | | | | |
|---|---|---|---|---|---|---|---|
| a. Commercially available PVM/MA copolymer under the trade name Gantrez® S-97 BF from Ashland b. Commercially available sodium alginate from Sinopharm Group Co.Ltd c. Commercially available xanthan gum under the trade name Keldent® Xanthan Gum from CP Kelco d. Commercially available carrageenan under the trade name GRINDSTED® Carrageenan CL110 from Danisco e. Commercially available acrylamide and sodium acrylamido-tertio-butyl sulfonate copolymer under the trade name Flopaam AN125 from SNF f. Commercially available acrylates copolymer under the trade name EPITEX™ 66 from Dow chemical company g. Commercially available titanium dioxide coated with calcium silicate from KOBO Products Inc h. Commercially available calcium silicate (CaSiO₃) under the trade name Sorbosil CA40 from P.Q.company | | | | | | | |

**TABLE 2**

| **Ingredient** | **Samples** | |
|---|---|---|
| | **8** | **9** |
| Glycerol | 75 | 74 |
| Xanthan gum | - | 1 |
| Titanium dioxide coated with calcium silicate^{g} | 15 | 15 |
| Calcium silicate^{h} | 10 | 10 |

### Methods

To evaluate deposition of particles on tooth surface, the test samples was mixed with water at a ratio of 5g to 10g de-ionized water (DI water) to make a slurry. Samples 1 and 8 comprising commercial calcium silicate and particles were used as controls for hydrous formulations and anhydrous formulations respectively.

HAP disks were cleaned and embedded in a silastic fixture. Then they were treated with different slurries via brushing following the same protocol. The HAP disks were brushed with the slurry under a tooth brushing machine equipped with toothbrushes. The load of the tooth brushing was 170g +/-5 g and the automatic brushing operated at a speed of 150 rpm. After brushing for 1 min, the HAP disks were rinsed with distilled water twice and soaked in simulated oral fluid (SOF) under the condition of a shaking water bath at 37°C and 60.0 rpm. After soaking for about 3 hours, the HAP disks were brushed with the slurry by machine using the same procedure as in the first step. The HAP disks were soaked in SOF for around 3 hours before brushed with the slurry again following the same procedure as in the first step. The HAP disks were kept in SOF overnight (>12 hours) in a shaking water bath at 37°C to mimic oral environment. Then the HAP disks were brushed with DI water by machine for 1 min and dried naturally. These steps are considered as a whole treatment cycle. The HAP disks were treated for one and three cycles.

The amount of titanium deposition was measured by X-ray fluorescence (XRF) using Axios wavelength dispersive XRF spectrometer. Higher XRF intensity indicates higher deposition of titanium containing particles on HAP disks.

Simulated oral fluid was made by combining the ingredients in Table 3:

**TABLE 3**

| Ingredient | Amount/g |
|---|---|
| NaCl | 16.07 |
| NaHCO₃ | 0.7 |
| KCl | 0.448 |
| K₂HPO₃*H₂O | 3.27 |
| MgCl₂*6H₂O | 0.622 |
| 1M HCl | 40ml |
| CaCl₂ | 0.1998 |
| Na₂SO₄ | 0.1434 |
| Buffer | Adjust pH to 7.0 |
| Water | Balance to 2L |

### Results

The results after one cycle and three cycles of treatment for hydrous formulations and anhydrous formulations are summarized in Table 4 and Table 5 respectively (error represents standard error for duplicate measurements).

**TABLE 4**

| XRF Intensity /kcps | **Samples** | | | | | | |
|---|---|---|---|---|---|---|---|
| | **1** | **2** | **3** | **4** | **5** | **6** | **7** |
| One cycle | 2.90±1.04 | 3.05±0.47 | 2.80±0.35 | 12.03±1.50 | 1.77±0.45 | 3.47±0.83 | 4.87±2.75 |
| Three cycles | 2.40±0.25 | 3.44±0.28 | 2.63±0.37 | 14.92±082 | 2.38±0.51 | 3.78±0.67 | 2.86±0.28 |

**TABLE 5**

| **XRF Intensity/kcps** | **Samples** | |
|---|---|---|
| | **8** | **9** |
| One cycle | 1.93±0.71 | 4.81±0.90 |
| Three cycles | 3.27±0.99 | 7.71±1.74 |

After one and three cycles of treatments, XRF measurement was used to determine the deposition of titanium on HAP disks. In hydrous formulations, it can be seen from the results that HAP disks treated with sample 4 comprising xanthan gum and calcium silicate showed significantly higher titanium deposition than those treated with other samples, which indicates significantly more particles were deposited on HAP disks. Similarly in anhydrous formulations, sample 9 comprising xanthan gum and calcium silicate behaved better in aiding particles deposition than sample 8 comprising only calcium silicate.

### Example 2

This example demonstrates the effect of particles concentration on the particles deposition efficacy. All ingredients are expressed by weight percent of the total formulation, and as level of active ingredient

**TABLE 6**

| Ingredient | Samples | | | | | |
|---|---|---|---|---|---|---|
| | **10** | **11** | **12** | **13** | **14** | **15** |
| Sorbitol | 45 | 45 | 45 | 45 | 45 | 45 |
| Water | 43.5 | 42 | 39 | 36 | 29 | 29 |
| Xanthan gum^{c} | 1 | 1 | 1 | 1 | 1 | 1 |
| Titanium dioxide | 0.5 | 2 | 5 | 8 | 15 | - |
| Titanium dioxide coated with calcium silicate^{g} | - | - | - | - | - | 15 |
| Calcium silicate^{h} | 10 | 10 | 10 | 10 | 10 | 10 |

### Methods

The same protocol was used to evaluate the deposition of particles on tooth surfaces as described in Example 1.

### Results

The results after one cycle and three cycles of treatment are summarized in Table 7 (error represents standard error for duplicate measurements).

**TABLE 7**

| XRF Intensity/kcps | Samples | | | | | |
|---|---|---|---|---|---|---|
| | **10** | **11** | **12** | **13** | **14** | **15** |
| One cycle | 0.04±0.07 | 0.65±0.09 | 1.76±0.22 | 3.87±0.25 | 3.75±0.41 | 8.82±0.66 |
| Three cycles | 0.42±0.10 | 1.21±0.07 | 3,15±0.16 | 4.34±0.17 | 6.00±0.65 | 15.39±0.80 |

After one cycle of treatment, HAP disks treated with sample 10 comprising 0.5% TiO₂ showed almost no titanium deposition, while the HAP disks treated with samples 13 and 14 comprising more than 5% of TiO₂ showed much higher titanium deposition. Moreover, sample 15 comprising composite particles behaved better in particles deposition than sample 14 comprising uncoated TiO₂ particles.

### Example 3

This example demonstrates the effect of xanthan gum concentration on the particles deposition efficacy. All ingredients are expressed by weight percent of the total formulation, and as level of active ingredient.

**TABLE 8**

| Ingredient | Samples | | | | | |
|---|---|---|---|---|---|---|
| | **16** | **17** | **18** | **19** | **20** | **21** |
| Sorbitol | 45 | 45 | 45 | 45 | 45 | 45 |
| Water | 30 | 29.9 | 29.8 | 29.7 | 29.5 | 29 |
| Xanthan gum^{c} | - | 0.1 | 0.2 | 0.3 | 0.5 | 1.0 |
| Titanium dioxide | 15 | 15 | 15 | 15 | 15 | 15 |
| Calcium silicate^{h} | 10 | 10 | 10 | 10 | 10 | 10 |

### Methods

The same protocol was used to evaluate the deposition of particles on tooth surfaces as described in Example 1.

### Results

The results after one cycle of treatment are summarized in Table 9 (error represents standard error for duplicate measurements).

**TABLE 9**

| XRF Intensity/kcps | Samples | | | | | |
|---|---|---|---|---|---|---|
| | **16** | **17** | **18** | **19** | **20** | **21** |
| One cycle | 0.92±0.11 | 1.10±0.14 | 1.38±0.12 | 1.34±0.20 | 2.22±0.23 | 5.83±1.76 |

It can be seen from the XRF results that the concentration of xanthan gum is important to the particles deposition efficacy. Sample 16 comprising no xanthan gum is used as control. Samples 17 to 19 comprising 0.3% xanthan gum or less showed comparable titanium deposition to sample 16, indicating low amount of xanthan gum did not aid the deposition of particles. Sample 20 and 21 comprising more than 0.3% xanthan gum behaved much better in aiding particles deposition compared to sample 16.

## Claims

1. An oral care composition comprising:
a) from 5 to 80% by weight of calcium silicate;
b) from 0.4% to 2% by weight of xanthan gum;
c) a benefit agent; and
d) a physiologically acceptable carrier;
wherein the benefit agent is selected from coloring agents, biomineralization agents, antibacterial agents or mixtures thereof and wherein the biomineralization agents is amorphous calcium phosphate, α-tricalcium phosphate, β-tricalcium phosphate; calcium carbonate, calcium deficient hydroxyapatite (Ca₉(HPO₄)(PO₄)₅OH), dicalcium phosphate (CaHPO₄), dicalcium phosphate dehydrate (CaHPO₄·2H₂O), hydroxyapatite (Ca₁₀(PO₄)₆(OH)₂), monocalcium phosphate monohydrate (Ca(H₂PO₄)₂·H₂O), octacalcium phosphate (Ca₈H₂(PO₄)₆·5H₂O) and tetracalcium phosphate (Ca₄(PO₄)₂O) or mixtures thereof.

2. The oral care composition according to claim 1, wherein the xanthan gum is present in an amount from 0.5 to 1.5%, preferably from 0.6 to 1.2% by weight of the composition.

3. The oral care composition according to claim 1 or claim 2, wherein the benefit agent is a particulate whitening agent.

4. The oral care composition according to claim 3, wherein the particulate whitening agent comprises a material having a refractive index ranging from 1.9 to 4.0.

5. The oral care composition according to claim 3, wherein the particulate whitening agent is a composite particle.

6. The oral care composition according to claim 5, wherein the composite particle comprises a first component core comprising a metal salt, preferably the metal is selected from zinc, titanium, zirconium or a mixture thereof, and a second component coating comprising the element calcium, and optionally, potassium, sodium, magnesium, aluminium or a mixture thereof.

7. The oral care composition according to claim 5 or claim 6, wherein the composite particle is titanium dioxide coated with calcium silicate.

8. The oral care composition according to any of the preceding claims, wherein the benefit agent is present in an amount from 0.25 to 60%, preferably from 1 to 30% by weight of the composition.

9. The oral care composition according to any of the preceding claim, wherein the relative weight ratio of calcium silicate to benefit agents ranges from 1:3 to 2:1.

10. The oral care composition according to any of the preceding claims, wherein the calcium silicate is present in an amount from 5 to 50%, preferably from 8 to 20% by weight of the composition.

11. The oral care composition according to any of the preceding claims, wherein the composition further comprises a phosphate source selected from trisodium phosphate, monosodium dihydrogen phosphate, disodium hydrogen phosphate, sodium pyrophosphate, tetrasodium pyrophosphate, sodium hexametaphosphate, tripotassium phosphate, monopotassium dihydrogen phosphate, dipotassium hydrogen phosphate or a mixture thereof, preferably trisodium phosphate, monosodium dihydrogen phosphate or mixtures thereof.

12. The oral care composition according to any of the preceding claims, wherein the composition is a monophase anhydrous composition.

13. The oral care composition according to claim 11, wherein the composition is a dual-phase composition comprising a calcium phase and a phosphate phase, wherein the calcium silicate is present in the calcium phase and the xanthan gum is present in either of the two phases.

14. Use of xanthan gum in the oral care compositions as claimed in any one of claims 1 to 13 for enhancing the deposition of calcium silicate and/or benefit agents on tooth surfaces; wherein the benefit agent is a particulate whitening agent or a pigment.

15. The use according to claim 14, wherein the pigment is violet or blue having a hue angle, h, in the CIELAB system of from 220 to 320 degrees.

## Patentansprüche

1. Mundpflegezusammensetzung, umfassend:
a) von 5 bis 80 Gewichts-% Calciumsilikat;
b) von 0,4 bis 2 Gewichts-% Xanthangummi;
c) ein Vorteilsmittel; und
d) einen physiologisch verträglichen Träger;
wobei das Vorteilsmittel unter Farbmitteln, Biomineralisierungsmitteln, antibakteriellen Mitteln oder Mischungen davon ausgewählt ist und wobei die Biomineralisierungsmittel amorphes Calciumphosphat, α-Tricalciumphosphat, β-Tricalciumphosphat, Calciumcarbonat, Calcium-defizienter Hydroxyapatit (Ca₉(HPO₄)(PO₄)₅OH, Dicalciumphosphat (CaHPO₄), Dicalciumphosphat-Dihydrat (CaHPO₄·2H₂O), Hydroxyapatit (Ca₁₀(PO₄)₆(OH)₂), Monocalciumphosphat-Monohydrat (Ca(H₂PO₄)₂·H₂O), Octacalciumphosphat (Ca₈H₂(PO₄)₆·5H₂O) und Tetracalciumphosphat (Ca₄(PO₄)₂O) oder Mischungen davon darstellen.

2. Mundpflegezusammensetzung nach Anspruch 1, wobei das Xanthangummi in einer Menge von 0,5 bis 1,5 Gewichts-%, vorzugsweise von 0,6 bis 1,2 Gewichts-% der Zusammensetzung vorliegt.

3. Mundpflegezusammensetzung nach Anspruch 1 oder Anspruch 2, wobei das Vorteilsmittel ein teilchenförmiges Aufhellungsmittel ist.

4. Mundpflegezusammensetzung nach Anspruch 3, wobei das teilchenförmige Aufhellungsmittel ein Material mit einem Brechungsindex, der zwischen 1,9 und 4,0 liegt, umfasst.

5. Mundpflegezusammensetzung nach Anspruch 3, wobei das teilchenförmige Aufhellungsmittel ein Verbundteilchen ist.

6. Mundpflegezusammensetzung nach Anspruch 5, wobei das Verbundteilchen einen Kern als erste Komponente, umfassend ein Metallsalz, wobei das Metall vorzugsweise aus Zink, Titan, Zirconium oder einer Mischung davon ausgewählt ist, und eine Beschichtung als zweite Komponente, umfassend das Element Calcium und optional Kalium, Natrium, Magnesium, Aluminium oder eine Mischung davon, umfasst.

7. Mundpflegezusammensetzung nach Anspruch 5 oder 6, wobei das Verbundteilchen ein mit Calciumsilikat beschichtetes Titandioxid ist.

8. Mundpflegezusammensetzung nach irgendeinem der vorhergehenden Ansprüche, wobei das Vorteilsmittel in einer Menge von 0,25 bis 60 Gewichts-%, vorzugsweise von 1 bis 30 Gewichts-% der Zusammensetzung vorliegt.

9. Mundpflegezusammensetzung nach irgendeinem vorhergehenden Anspruch, wobei das relative Gewichtsverhältnis von Calciumsilikat zu Vorteilsmitteln in dem Bereich von 1:3 bis 2:1 liegt.

10. Mundpflegezusammensetzung nach irgendeinem der vorhergehenden Ansprüche, wobei das Calciumsilikat in einer Menge von 5 bis 50 Gewichts-%, vorzugsweise von 8 bis 20 Gewichts-% der Zusammensetzung vorliegt.

11. Mundpflegezusammensetzung nach irgendeinem der vorhergehenden Ansprüche, wobei die Zusammensetzung ferner eine Phosphatquelle umfasst, ausgewählt aus Trinatriumphosphat, Mononatriumdihydrogenphosphat, Dinatriumhydrogenphosphat, Natriumpyrophosphat, Tetranatriumpyrophosphat, Natriumhexametaphosphat, Trikaliumphosphat, Monokaliumdihydrogenphosphat, Dikaliumhydrogenphosphat oder einer Mischung davon, vorzugswiese Trinatriumphosphat, Mononatriumdihydrogenphosphat oder Mischungen davon.

12. Mundpflegezusammensetzung nach irgendeinem der vorhergehenden Ansprüche, wobei die Zusammensetzung eine einphasige wasserfreie Zusammensetzung ist.

13. Mundpflegezusammensetzung nach Anspruch 11, wobei die Zusammensetzung eine zweiphasige Zusammensetzung, umfassend eine Calcium-Phase und eine Phosphat-Phase, ist, wobei das Calciumsilikat in der Calcium-Phase und das Xanthangummi in einer der zwei Phasen vorliegt.

14. Verwendung von Xanthangummi in den Mundpflegezusammensetzungen, wie in irgendeinem der Ansprüche 1 bis 13 beansprucht, zum Verbessern der Ablagerung von Calciumsilikat und/oder Vorteilsmitteln auf Zahnoberflächen, wobei das Vorteilsmittel ein teilchenförmiges Aufhellungsmittel oder ein Pigment ist.

15. Verwendung nach Anspruch 14, wobei das Pigment violett oder blau ist und einen Farbtonwinkel h in dem CIELAB-System von 220 bis 320 Grad aufweist.

## Revendications

1. Composition pour le soin oral comprenant :
a) de 5 à 80 % en masse de silicate de calcium ;
b) de 0,4 % à 2 % en masse de gomme xanthane ;
c) un agent bénéfique ; et
d) un support physiologiquement acceptable ;
dans laquelle l'agent bénéfique est choisi parmi des agents colorants, des agents de biominéralisation, des agents antibactériens ou des mélanges de ceux-ci et dans laquelle les agents de biominéralisation sont du phosphate de calcium amorphe, phosphate d'a-tricalcium, phosphate de β-tricalcium ; carbonate de calcium, hydroxyapatite déficiente en calcium (Ca₉(HPO₄)(PO₄)₅OH), phosphate de dicalcium (CaHPO₄), dihydrate de phosphate de dicalcium (CaHPO₄.2H₂O), hydroxyapatite (Ca₁₀(PO₄)₆(OH)₂), monohydrate de phosphate de monocalcium (Ca(H₂PO₄)₂.H₂O), phosphate d'octacalcium (Ca₈H₂(PO₄)₆.5H₂O) et phosphate de tétracalcium (Ca₄(PO₄)₂O) ou des mélanges de ceux-ci.

2. Composition pour le soin oral selon la revendication 1, dans laquelle la gomme xanthane est présente dans une quantité de 0,5 à 1,5 %, de préférence de 0,6 à 1,2 % en masse de la composition.

3. Composition pour le soin oral selon la revendication 1 ou la revendication 2, dans laquelle l'agent bénéfique est un agent blanchissant particulaire.

4. Composition pour le soin oral selon la revendication 3, dans laquelle l'agent blanchissant particulaire comprend un matériau présentant un indice de réfraction de 1,9 à 4,0.

5. Composition pour le soin oral selon la revendication 3, dans laquelle l'agent blanchissant particulaire est une particule composite.

6. Composition pour le soin oral selon la revendication 5, dans laquelle la particule composite comprend un noyau de premier constituant comprenant un sel de métal, le métal est de préférence choisi parmi le zinc, titane, zirconium ou un mélange de ceux-ci, et un revêtement de second constituant comprenant le calcium élément, et éventuellement du potassium, sodium, magnésium, aluminium ou un mélange de ceux-ci.

7. Composition pour le soin oral selon la revendication 5 ou la revendication 6, dans laquelle la particule composite est du dioxyde de titane revêtu avec du silicate de calcium.

8. Composition pour le soin oral selon l'une quelconque des revendications précédentes, dans laquelle l'agent bénéfique est présent dans une quantité de 0,25 à 60 %, de préférence de 1 à 30 % en masse de la composition.

9. Composition pour le soin oral selon l'une quelconque des revendications précédentes, dans laquelle le rapport massique relatif de silicate de calcium aux agents bénéfiques est de 1:3 à 2:1.

10. Composition pour le soin oral selon l'une quelconque des revendications précédentes, dans laquelle le silicate de calcium est présent dans une quantité de 5 à 50 %, de préférence de 8 à 20 % en masse de la composition.

11. Composition pour le soin oral selon l'une quelconque des revendications précédentes, dans laquelle la composition comprend de plus une source de phosphate choisie parmi le phosphate de trisodium, dihydrogénophosphate de monosodium, hydrogénophosphate de disodium, pyrophosphate de sodium, pyrophosphate de tétrasodium, hexamétaphosphate de sodium, phosphate de tripotassium, dihydrogénophosphate de monopotassium, hydrogénophosphate de dipotassium ou un mélange de ceux-ci, de préférence phosphate de trisodium, dihydrogénophosphate de monosodium ou des mélanges de ceux-ci.

12. Composition pour le soin oral selon l'une quelconque des revendications précédentes, dans laquelle la composition est une composition anhydre de monophase.

13. Composition pour le soin oral selon la revendication 11, dans laquelle la composition est une composition de phase double comprenant une phase de calcium et une phase de phosphate, dans laquelle le silicate de calcium est présent dans la phase de calcium et la gomme xanthane est présente dans l'une des deux phases.

14. Utilisation de gomme xanthane dans les compositions de soin oral selon l'une quelconque des revendications 1 à 13 pour promouvoir le dépôt de silicate de calcium et/ou d'agents bénéfiques sur des surfaces de dents ; dans laquelle l'agent bénéfique est un agent blanchissant particulaire ou un pigment.

15. Utilisation selon la revendication 14, dans laquelle le pigment est violet ou bleu ayant un angle de teinte, h, dans le système CIELAB de 220 à 320 degrés.
